# EUROPÄISCHE PATENTSCHRIFT

(11) **EP 2 114 544 B1**
(45) Veröffentlichungstag und Bekanntmachung des Hinweises auf die Patenterteilung: **03.08.2011**
(21) Anmeldenummer: 08708109.7
(22) Anmeldetag: 23.01.2008
(51) Int. Cl.: B01D 3/14, C07D 323/06

(54) **VERFAHREN ZUR ABTRENNUNG VON TRIOXAN AUS EINEM TRIOXAN/FORMALDEHYD/WASSER-GEMISCH MITTELS DRUCKWECHSEL-REKTIFIKATION**
METHOD FOR SEPARATING TRIOXANE FROM A TRIOXANE/FORMALDEHYDE/WATER MIXTURE BY MEANS OF PRESSURE CHANGE RECTIFICATION
PROCÉDÉ DE SÉPARATION DE TRIOXANE D'UN MÉLANGE TRIOXANE/FORMALDÉHYDE/EAU PAR RECTIFICATION À ÉCHANGE DE PRESSION

(30) Priorität: 25.01.2007 EP 07101198
(43) Veröffentlichungstag der Anmeldung: 11.11.2009
(73) Patentinhaber: BASF SE, 67056 Ludwigshafen (DE)
(72) Erfinder: Lang, Neven, 68163 Mannheim (DE); Thiel, Joachim, 67435 Neustadt (DE); Stroefer, Eckhard, 68163 Mannheim (DE); Kirschbaum, Julia, 67063 Ludwigshafen (DE); Siegert, Markus, 69126 Heidelberg (DE)
(86) Internationale Anmeldenummer: PCT/EP2008/050745
(87) Internationale Veröffentlichungsnummer: WO 2008/090169

(56) Entgegenhaltungen:
- WO-A-2005/063353
- WO-A-2005/063733

## Beschreibung

Die Erfindung betrifft ein Verfahren zur Abtrennung von Trioxan aus einem Trioxan/Formaldehyd/Wasser-Gemisch sowie ein Verfahren zur Herstellung von Trioxan.

Trioxan wird in der Regel durch Destillation von wässriger Formaldehydlösung in Gegenwart saurer Katalysatoren hergestellt. Dem Formaldehyd und Wasser enthaltenden Destillat wird anschließend das Trioxan durch Extraktion mit halogenierten Kohlenwasserstoffen, wie Methylenchlorid oder 1,2-Dichlorethan, oder anderen, mit Wasser nicht mischbaren Lösungsmitteln entzogen.

DE-A 1 668 867 beschreibt ein Verfahren zur Abtrennung von Trioxan aus Wasser, Formaldehyd und Trioxan enthaltenden Gemischen durch Extraktion mit einem organischen Lösungsmittel. Dabei wird eine aus zwei Teilstrecken bestehende Extraktionsstrecke an einem Ende mit einem üblichen organischen, mit Wasser praktisch nicht mischbaren Extraktionsmittel für Trioxan beschickt, am anderen Ende mit Wasser. Zwischen den beiden Teilstrecken wird das zu trennende Destillat der Trioxan-Synthese zugeführt. Auf der Seite der Lösungsmittelzuführung wird dann eine wässrige Formaldehydlösung und auf der Seite der Wasserzuführung eine praktisch formaldehydfreie Lösung von Trioxan in dem Lösungsmittel erhalten. In einem Beispiel wird das bei der Trioxan-Synthese entstandene Destillat aus 40 Gew.-% Wasser, 35 Gew.-% Trioxan und 25 Gew.-% Formaldehyd in den Mittelteil einer Pulsationskolonne eindosiert, am oberen Kolonnenende Methylenchlorid und am unteren Kolonnenende Wasser zugeführt. Dabei wird am unteren Kolonnenende eine etwa 25 gew.-%ige Lösung von Trioxan in Methylenchlorid und am oberen Kolonnenende eine etwa 30 gew.-%ige wässrige Formaldehydlösung erhalten.

Nachteil dieser Verfahrensweise ist der Anfall an Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrstoffverordnung), deren Handhabung besondere Vorsichtsmaßnahmen erfordert.

DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in Trioxan und ein azeotropes Gemisch aus Trioxan, Wasser und Formaldehyd trennt. In dem Beispiel wird ein wässri-B06/0906PC ges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trioxan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt.

Nachteilig an dieser Verfahrensweise sind die sehr hohen Investitionen für die Pervaporationseinheit.

Ein weikres verfahren, bei dem Trioxan als Hanpkomponente zurück-geführt wird, ist in WO 2005/063733 beschrieben.

Aufgabe der Erfindung ist es, ein Verfahren zur Abtrennung von Trioxan aus azeotropen Trioxan/Formaldehyd/Wasser-Gemischen bereitzustellen, welches ohne die Extraktionsschritte oder Pervaporationsschritte des Standes der Technik auskommt.

Gelöst wird diese Aufgabe durch ein Verfahren zur Abtrennung von Trioxan aus einem Einsatzstrom I aus Formaldehyd, Trioxan und Wasser, bei dem
a) ein Einsatzstrom I, der als Hauptkomponente Formaldehyd und als Nebenkomponenten Trioxan und Wasser enthält, bereitgestellt wird,
b) der Einsatzstrom I, ein Rückführstrom V und ein Rückführstrom VII, der als Hauptkomponente Formaldehyd und als Nebenkomponenten Wasser und Trioxan enthält, in eine erste Destillationsstufe eingespeist und bei einem Druck von 0,1 bis 2,5 bar destilliert werden, wobei ein Strom II, der als Hauptkomponente Formaldehyd und als Nebenkomponente Wasser enthält, und ein Strom III, der als Hauptkomponente Trioxan und als Nebenkomponenten Wasser und Formaldehyd enthält, und ein Strom X, der Wasser, Trioxan und Formaldehyd enthält, erhalten werden,
c) der Strom III, gegebenenfalls nach Abtrennung von Leichtsiedern aus dem Strom III in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom IV, der im Wesentlichen aus Trioxan besteht, und der Rückführstrom V, der als Hauptkomponente Trioxan und als Nebenkomponenten Wasser und Formaldehyd enthält, erhalten wird,
d) der Strom X und gegebenenfalls ein Strom IX, der als Hauptkomponente Wasser enthält, in eine dritte Destillationsstufe eingespeist und bei einem Druck von 1 bis 10 bar destilliert wird, wobei ein Strom VI, der im Wesentlichen aus Wasser besteht, und ein Rückführstrom VII, der Formaldehyd und Wasser und Trioxan enthält, erhalten werden.

Die Hauptkomponente ist die Komponente mit dem größeren beziehungsweise größten Massenanteil an dem betreffenden Gemisch. Vorzugsweise beträgt der Massenanteil der Hauptkomponente an dem jeweiligen Gemisch mindestens 40 Gew.-%. Ein Strom "besteht im Wesentlichen aus" einer oder mehreren Komponenten, wenn er zu mindestens 90 Gew.-% aus dieser beziehungsweise diesen Komponenten besteht.

Es ist bekannt, dass Trioxan, Formaldehyd und Wasser ein ternäres Azeotrop bilden, welches bei einem Druck von 1 bar die Zusammensetzung 69,5 Gew.-% Trioxan, 5,4 Gew.-% Formaldehyd und 25,1 Gew.-% Wasser aufweist.

Erfindungsgemäß wird dieses Azeotrop durch Druckwechseldestillation umgangen, bei dem eine erste und eine zweite Destillation bei verschiedenen Drücken durchgeführt werden. In einer ersten Destillationskolonne, welche bei niedrigerem Druck betrieben wird, wird das Ausgangsgemisch in ein Trioxan/Wasser-Gemisch mit geringem Formaldehyd-Gehalt III und ein im Wesentlichen trioxanfreies Formaldehyd/WasserGemisch II aufgetrennt. Das Formaldehyd/Wasser-Gemisch II kann in die Trioxan-Synthese zurückgeführt werden. In einer zweiten, bei höherem Druck betriebenen Destillationskolonne wird das erhaltene Trioxan/Formaldehyd/Wasser-Gemisch III in reines Trioxan und ein Trioxan/Formaldehyd/Wasser-Gemisch V mit niedrigerem Trioxan-Gehalt aufgetrennt. Das Gemisch V wird in die erste Destillationskolonne zurückgeführt. Erfindungsgemäß wird weiterhin in der ersten Destillationskolonne als Seitenabzugsstrom X ein stark wasserhaltiges Gemisch gewonnen, aus dem in einer dritten Destillationskolonne im Wesentlichen reines Wasser VI abgetrennt und ein wasserärmeres Trioxan/Formaldehyd/Wasser-Gemisch VII gewonnen wird. Dieses Gemisch VII wird in die erste Destillationskolonne zurückgeführt. Vorzugsweise wird ein wasserhaltiger Strom IX, der bei der Aufkonzentrierung von wässriger Formaldehydlösung erhalten wird, ebenfalls in die dritte Destillationskolonne eingespeist.

Als Destillationskolonnen sind beliebige Destillationskolonnen wie Packungs- und Bodenkolonnen geeignet. Diese können beliebige Einbauten, Packungen oder Füllkörperschüttungen enthalten.

Der Druck in der zweiten Destillationsstufe ist um 0,1 bis 15 bar höher als der Druck in der ersten Destillationsstufe. Vorzugsweise beträgt diese Druckdifferenz 1,0 bis 10 bar, besonders bevorzugt 1,5 bis 5 bar.

Alle Druckangaben beziehen sich auf den Druck am Kopf der jeweiligen Kolonne.

Die erste Destillationsstufe wird bei einem Druck von 0,1 bis 2,5 bar, vorzugsweise 0,25 bis 1,5 bar durchgeführt. Die erste Destillationsstufe wird im Allgemeinen in einer Destillationskolonne mit mindestens 2, vorzugsweise 2 bis 50, besonders bevorzugt 4 bis 25 theoretischen Stufen durchgeführt. Im Allgemeinen umfasst der Abtriebsteil dieser Kolonne mindestens 25 %, vorzugsweise 50 bis 90 % der theoretischen Stufen dieser Kolonne.

Der Einspeisungsstrom I enthält im Allgemeinen 40 bis 80 Gew.-% Formaldehyd, 20 bis 59 Gew.-% Wasser und 1,0 bis 30 Gew.-% Trioxan. Der Einspeisungsstrom I wird vorzugsweise dampfförmig in den Sumpf der ersten Destillationskolonne eingespeist.

Der Strom II, der im Allgemeinen als Sumpfabzugsstrom der ersten Destillationskolonne erhalten wird, enthält im Allgemeinen weniger als 5 Gew.-%, bevorzugt weniger als 2 Gew.-% Trioxan, besonders bevorzugt weniger als 1 Gew.-% Trioxan. Beispielsweise setzt sich der Strom II wie folgt zusammen: 55 bis 85 Gew.-% Formaldehyd, 15 bis 45 Gew.-% Wasser und 0 bis 5 Gew.-% Trioxan. Der Strom III, der im Allgemeinen als Kopfabzugsstrom der ersten Destillationskolonne erhalten wird, enthält im Allgemeinen mehr als 60 Gew.-%, vorzugsweise mehr als 63 Gew.-%, besonders bevorzugt mehr als 65 Gew.-% Trioxan. Beispielsweise setzt sich der Strom III wie folgt zusammen: 3 bis 20 Gew.-% Formaldehyd, 10 bis 30 Gew.-% Wasser und 60 bis 75 Gew.-% Trioxan. Der Strom X, der als Seitenabzugsstrom der ersten Destillationskolonne erhalten wird, enthält Wasser, Formaldehyd und Trioxan, wobei im Allgemeinen Wasser oder Formaldehyd die Hauptkomponente ist. Beispielsweise setzt sich der Strom X wie folgt zusammen: 10 bis 50 Gew.-% Formaldehyd, 10 bis 50 Gew.-% Wasser und 3 bis 40 Gew.-% Trioxan.

Der Strom II wird vorzugsweise in die Trioxan-Synthese zurückgeführt.

Die Ströme I, III, V und VII können noch bis zu 15 Gew.-% Leichtsieder enthalten. Übliche Leichtsieder, die bei der Trioxan-Synthese und der nachfolgenden destillativen Trennung gebildet werden können, sind Methylformiat, Methylal, Dimethoxydimethylether, Methanol, Ameisensäure sowie weitere Halb- und Vollacetale. Zur Abtrennung dieser Leichtsieder kann optional zwischen der ersten und der zweiten Destillationsstufe eine Leichtsieder-Abtrennstufe durchgeführt werden. Dabei werden die Leichtsieder vorzugsweise über den Kopf einer Leichtsieder-Abtrennkolonne, welche im Allgemeinen bei einem Druck von 0,1 bis 5 bar, vorzugsweise bei einem Druck von 1,0 bis 2,5 bar betrieben wird, abgetrennt. Im Allgemeinen weist die Leichtsieder-Abtrennkolonne mindestens 2 theoretische Stufen, vorzugsweise 15 bis 50 theoretische Stufen auf. Im Allgemeinen umfasst der Abtriebsteil dieser Kolonne 25 bis 90 %, vorzugsweise 50 bis 75 % der theoretischen Stufen dieser Kolonne. Der Gehalt der gegenüber Trioxan leichter siedenden Komponenten im Sumpfaustrag der Leichtsieder-Abtrennkolonne beträgt im Allgemeinen weniger als 5 Gew.-%, bevorzugt weniger als 2,5 Gew.-%, besonders bevorzugt weniger als 1,5 Gew.-%.

Im Allgemeinen wird eine Leichtsieder-Abtrennung durchgeführt.

Der Strom III wird in einer zweiten Destillationsstufe bei einem Druck von 0,2 bis 17,5 bar in einen Strom IV aus im Wesentlichen reinem Trioxan und einen Strom V, der als Hauptkomponente Trioxan und daneben Wasser und Formaldehyd enthält, aufgetrennt. Diese zweite Destillationsstufe wird vorzugsweise bei 2,5 bis 10 bar durchgeführt. Im Allgemeinen wird diese zweite Destillationsstufe in einer Destillationskolonne mit mindestens 2 theoretischen Böden, vorzugsweise 10 bis 50 theoretischen Böden, durchgeführt, wobei der Strom IV als Sumpfabzugsstrom oder als Seitenabzugsstrom im Abtriebsteil der Kolonne anfällt und der Strom V als Kopfabzugsstrom anfällt. Im Allgemeinen umfasst der Abtriebsteil dieser Destillationskolonne 25 bis 90 %, vorzugsweise 50 bis 75 % der theoretischen Stufen dieser Kolonne.

Im Allgemeinen enthält der Strom IV 95 bis 100 Gew.-%, vorzugsweise 99 bis 100 Gew.-% Trioxan und 0 bis 5 Gew.-%, vorzugsweise 0 bis 1 Gew.-% Wasser und Nebenkomponenten. Nebenkomponenten sind insbesondere die oben genannten Leichtsieder, aber auch höher als Trioxan siedende Komponenten. Besonders bevorzugt ist der Gehalt an Wasser und Nebenkomponenten im Trioxan-Strom IV < 0,1 %. Er kann sogar < 0,01 % sein. Der Strom V enthält beispielsweise 5 bis 20 Gew.-% Formaldehyd, 15 bis 35 Gew.-% Wasser und 50 bis 75 Gew.-% Trioxan.

Der Strom X und gegebenenfalls ein wasserhaltiger Strom IX werden in einer dritten Destillationsstufe bei einem Druck von 1 bis 10 bar in einen Strom VI, der im Wesentlichen aus Wasser besteht, und einen Rückführstrom VII, der als Hauptkomponente Trioxan und daneben Wasser und Formaldehyd enthält, aufgetrennt. Der wasserhaltige Strom IX wird gegebenenfalls als Brüdenabzugsstrom einer Formaldehyd-Aufkonzentrierungseinheit, die als Verdampfer ausgestaltet ist, gewonnen und enthält beispielsweise 70 bis 97 Gew.-% Wasser und 3 bis 30 Gew.-% Formaldehyd. Vorzugsweise wird die dritte Destillationsstufe bei einem Druck von 2,5 bis 8 bar durchgeführt. Im Allgemeinen wird die dritte Destillationsstufe in einer Destillationskolonne mit mindestens 2 theoretischen Böden, vorzugsweise 10 bis 50 theoretischen Böden, durchgeführt, wobei der Wasserstrom VI als Sumpfabzugsstrom oder als Seitenabzugsstrom der Kolonne und der Rückführstrom VII als Kopfabzugsstrom erhalten werden. Der Strom X wird vorzugsweise im oberen Bereich der Kolonne, beispielsweise im Bereich des obersten Drittels der theoretischen Böden der Kolonne, und der Strom IX im mittleren Bereich der Kolonne, beispielsweise im Bereich des mittleren Drittels der theoretischen Böden der Kolonne, zugegeben.

Der Wasserstrom VI besteht vorzugsweise zu mehr als 95 Gew.-%, besonders bevorzugt zu mehr als 97 Gew.-% aus Wasser. Beispielsweise enthält der Strom VI 98 bis 100 Gew.-% Wasser, 0 bis 1 Gew.-% Formaldehyd und 0 bis 1 Gew.-% Nebenkomponenten.

Der Strom VII enthält beispielsweise 10 bis 55 Gew.-% Formaldehyd, 5 bis 50 Gew.-% Wasser und 5 bis 55 Gew.-% Trioxan.

Der Strom VII kann teilweise oder vollständig vor die erste Destillationsstufe zurückgeführt werden, vorzugsweise wird er im Wesentlichen vollständig in die erste Destillationsstufe zurückgeführt. Dabei kann er mit dem Rückführstrom V gemischt oder getrennt von diesem der ersten Destillationskolonne zugeführt werden.

Gegenstand der vorliegenden Erfindung ist auch ein Verfahren zur Herstellung von Trioxan aus einer wässrigen Formaldehydlösung, bei dem der Formaldehyd, Trioxan und Wasser enthaltende Einsatzstrom I in einer vorgelagerten Trioxan-Synthesestufe aus einer wässrigen Formaldehydlösung hergestellt wird und anschließend aus dem Strom I wie vorstehend beschrieben Trioxan abgetrennt wird. Alternativ dazu können die Trioxan-Synthese und die erste Destillationsstufe in einer Reaktivdestillation vereinigt werden.

In einer Ausführungsform des erfindungsgemäßen Verfahrens wird ein Strom XI aus einer wässrigen Formaldehydlösung einer vorgelagerten Trioxan-Synthesestufe zugeführt und in Gegenwart saurer homogen oder heterogen vorliegender Katalysatoren wie Ionenaustauscherharze, Zeolithe, Schwefelsäure und p-Toluolsulfonsäure bei einer Temperatur von im Allgemeinen 70 bis 130 °C umgesetzt. Dabei kann in einer Destillationskolonne oder einem Verdampfer (Reaktivverdampfer) gearbeitet werden. Das Produktgemisch aus Trioxan/Formaldehyd und Wasser fällt dann als dampfförmiger Brüdenabzugsstrom des Verdampfers beziehungsweise als Kopfabzugsstrom am Kopf der Kolonne an. Die Trioxan-Synthesestufe kann auch in einem Festbett- oder Fließbettreaktor an einem heterogenen Katalysator, z. B. einem Ionenaustauscherharz oder Zeolith, durchgeführt werden.

In einer weiteren Ausführungsform des erfindungsgemäßen Verfahrens werden die Trioxan-Synthesestufe und die erste Destillationsstufe als Reaktivdestillation in einer Reaktionskolonne durchgeführt. Diese kann im Abtriebsteil ein Katalysator-Festbett aus einem heterogenen sauren Katalysator enthalten. Alternativ kann die Reaktivdestillation auch in Gegenwart eines homogenen Katalysators durchgeführt werden, wobei der saure Katalysator zusammen mit der wässrigen Formaldehyd-Lösung im Kolonnensumpf vorliegt.

Im Allgemeinen enthält die wässrige Formaldehydlösung, die der Trioxan-Synthesestufe zugeführt wird, 30 bis 85 Gew.-% Formaldehyd und 15 bis 70 Gew.-% Wasser. Diese Lösung kann in einem vorgelagerten Aufkonzentrierungsschritt aus einer wässrigen Formaldehydlösung mit niedrigerer Formaldehyd-Konzentration erhalten werden. Der Aufkonzentrierungsschritt kann beispielsweise in einem Verdampfer, vorzugsweise einem Fallfilmverdampfer, durchgeführt werden.

Der vorgelagerte Aufkonzentrierungsschritt kann beispielsweise wie in DE-A 199 25 870 beschrieben durchgeführt werden.

In einer Ausführungsform des erfindungsgemäßen Verfahrens wird ein Strom VIII einer wässrigen Formaldehydlösung in einem Verdampfer, vorzugsweise einem Fallfilmverdampfer, aufkonzentriert, wobei der Strom XI aus wässriger Formaldehydlösung mit höherer Formaldehyd-Konzentration erhalten wird. Der Brüdenabzugsstrom des Verdampfers, welcher stark an Formaldehyd abgereichert ist, wird als wasserhaltiger Strom IX in die dritte Destillationsstufe eingespeist. Strom VIII enthält beispielsweise 40 bis 60 Gew.-% Formaldehyd und 40 bis 60 Gew.-% Wasser. Der aufkonzentrierte Strom XI enthält beispielsweise 55 bis 80 Gew.-% Formaldehyd und 20 bis 45 Gew.-% Wasser. Der formaldehydarme Brüdenabzugsstrom IX enthält beispielsweise 10 bis 25 Gew.-% Formaldehyd und 75 bis 90 Gew.-% Wasser.

Das erhaltene Rein-Trioxan, dessen Reinheit > 99 Gew.-%, > 99,9 Gew.-% oder sogar > 99.99 Gew.-% betragen kann, wird vorzugsweise zur Herstellung von Polyoxymethylen (POM), Polyoxymethylenderivaten wie Polyoxymethylendimethylether (POMDME) und Diaminodiphenylmethan (MDA) verwendet.

Die Erfindung wird nachfolgend mit Bezugnahme auf die Zeichnung näher erläutert.

Es zeigt:

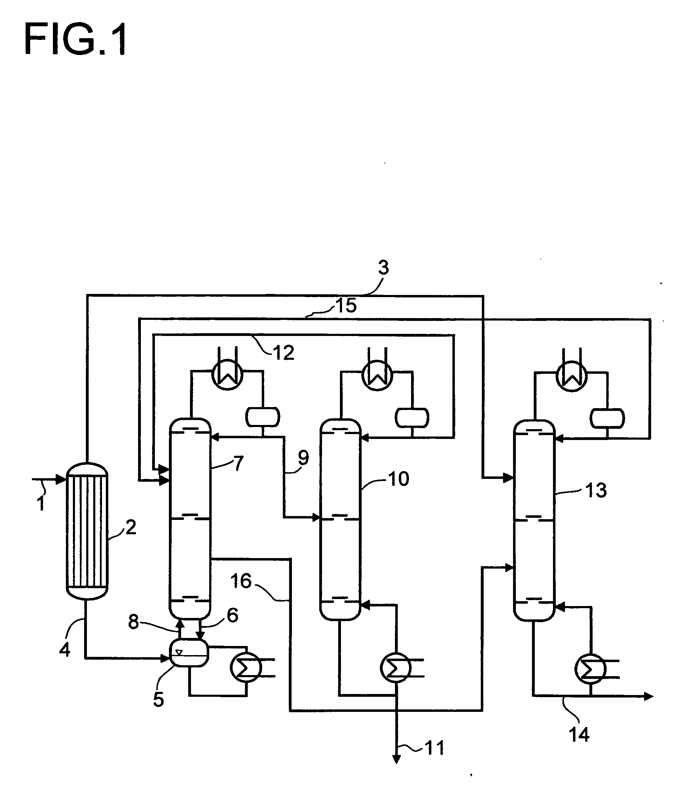

- Figur 1: beispielhaft eine Ausführungsform des erfindungsgemäßen Verfahrens.

Eine wässrige Formaldehydlösung **1** (Strom VIII) wird dem Verdampfer **2,** beispielsweise einem Dünnschichtverdampfer, Fallfilmverdampfer oder Wendelrohrverdampfer, zugeführt. Als Brüdenabzugsstrom **3** (Strom IX) des Verdampfers wird eine an Formaldehyd abgereicherte wässrige Lösung, als Sumpfabzugsstrom **4** (Strom XI) des Verdampfers eine formaldehydreiche wässrige Lösung erhalten. Diese wird mit dem formaldehydreichen Sumpfabzugsstrom **8** (Strom II) der ersten Destillationskolonne **7** dem Trioxan-Synthesereaktor **5,** der als Verdampfer ausgebildet ist, zugeführt. Das den Trioxan-Synthesereaktor verlassende dampfförmige Trioxan/Formaldehyd/Wasser-Gemisch **6** (Strom I) wird dem Sumpf der ersten Destillationskolonne **7** zugeführt. Der trioxanreiche Kopfabzugsstrom **15** (Strom VII) der dritten Destillationskolonne **13** wird der Destillationskolonne **7** in der Nähe des Kolonnenkopfes zugeführt. Der Destillationskolonne **7** wird ein Formaldehyd/Wasser-Strom **8** (Strom II) als Sumpfabzugsstrom, ein wasserarmer Formaldehyd/Wasser/Trioxan-Strom **9** (Strom III) als Kopfabzugsstrom und ein wasserreicher Formaldehyd/Wasser/Trioxan-Strom **16** als Seitenabzugsstrom entnommen. Strom **8** wird zusammen mit dem Strom **4** in den Reaktor **5** zurückgeführt. Der wasserarme Formaldehyd/Wasser/Trioxan-Strom **9** wird der Destillationskolonne **10** zugeführt und dort in einen Sumpfabzugsstrom **11** (Strom IV) aus im Wesentlichen reinem Trioxan und einen Kopfabzugsstrom **12** (Strom V), der überwiegend Trioxan und daneben Wasser und Formaldehyd enthält, aufgetrennt. Der Strom **12** wird in die erste Destillationskolonne zurückgeführt. Der wasserreiche Formaldehyd/Wasser/Trioxan-Strom **16** und der formaldehydarme wässrige Brüdenabzugsstrom **3** (Strom IX) des Verdampfers **2** werden der dritten Destillationskolonne zugeführt und dort in einen Strom **14** (Strom VI), der im Wesentlichen aus Wasser besteht und ausgeschleust wird, und den Rückführstrom **15** (Strom VII), der überwiegend Formaldehyd und daneben Wasser und Trioxan enthält, aufgetrennt.

### Beispiel

Bei der rechnerischen Simulation des in der Figur dargestellten Verfahrens wurden Stoffströme **4, 9, 11, 12, 3, 14, 15 und 16** der in den Tabellen angegebenen Zusammensetzungen erhalten. Dabei wurden folgende Parameter gewählt: Die erste Destillationsstufe wird bei einem Druck von 0,7 bar in einer Kolonne **7** mit 10 theoretischen Böden durchgeführt. Das Rücklaufverhältnis beträgt 0,8, die Kopftemperatur 80 °C und die Sumpftemperatur 94 °C. Die zweite Destillationsstufe wird bei einem Druck von 4,0 bar in einer Kolonne **10** mit 40 theoretischen Böden durchgeführt. Das Rücklaufverhältnis beträgt 0,5, die Kopftemperatur 146 °C und die Sumpftemperatur 181 °C. Der Zulauf **9** befindet sich auf Höhe des 35. theoretischen Bodens. Die dritte Destillationsstufe wird bei einem Druck von 6,0 bar in einer Kolonne **13** mit 10 theoretischen Böden durchgeführt. Das Rücklaufverhältnis beträgt 1,5, die Kopftemperatur 146 °C und die Sumpftemperatur 160 °C. Der Zulauf **3** befindet sich auf Höhe des 8. theoretischen Bodens.

| Strom | 4 (XI) | 9 (III) | 11 (IV) | 12 (V) | 3 (IX) | 14 (VI) | 15 (VII) | 16 (X) |
|---|---|---|---|---|---|---|---|---|
| Mengenstrom [kg/h] | 4,1 | 11,9 | 3 | 9,0 | 2,0 | 3,1 | 8,3 | 9,5 |
| Formaldehyd [Gew.-%] | 65,0 | 8,5 | < 1 | 11,3 | 15,3 | < 1 | 52,2 | 42,7 |
| Wasser [Gew.-] | 35,0 | 21,5 | < 1 | 28,7 | 84,7 | > 99 | 22,6 | 35,2 |
| Trioxan [Gew.-%] | 0 | 70,0 | > 99 | 60,0 | 0 | 0 | 25,2 | 22,1 |

## Patentansprüche

1. Verfahren zur Abtrennung von Trioxan aus einem Einsatzstrom I aus Formaldehyd, Trioxan und Wasser, bei dem
a) ein Einsatzstrom I, der als Hauptkomponente Formaldehyd und als Nebenkomponenten Trioxan und Wasser enthält, bereitgestellt wird,
b) der Einsatzstrom I, ein Rückführstrom V und ein Rückführstrom VII, der als Hauptkomponente Formaldehyd und als Nebenkomponenten Wasser und Trioxan enthält, in eine erste Destillationsstufe eingespeist und bei einem Druck von 0,1 bis 2,5 bar destilliert werden, wobei ein Strom II, der als Hauptkomponente Formaldehyd und als Nebenkomponente Wasser enthält, und ein Strom III, der als Hauptkomponente Trioxan und als Nebenkomponenten Wasser und Formaldehyd enthält, und ein Strom X, der Wasser, Trioxan und Formaldehyd enthält, erhalten werden,
c) der Strom III, gegebenenfalls nach Abtrennung von Leichtsiedern aus dem Strom III in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom IV, der im Wesentlichen aus Trioxan besteht, und der Rückführstrom V, der als Hauptkomponente Trioxan und als Nebenkomponenten Wasser und Formaldehyd enthält, erhalten wird,
d) der Strom X und gegebenenfalls ein Strom IX, der als Hauptkomponente Wasser enthält, in eine dritte Destillationsstufe eingespeist und bei einem Druck von 1 bis 10 bar destilliert wird, wobei ein Strom VI, der im Wesentlichen aus Wasser besteht, und ein Rückführstrom VII, der als Hauptkomponente Formaldehyd und als Nebenkomponenten Wasser und Trioxan enthält, erhalten werden.

2. Verfahren nach Anspruch 1, **dadurch gekennzeichnet, dass** der Druck in der zweiten Destillationsstufe um 1,0 bis 10 bar höher als der Druck in der ersten Destillationsstufe ist.

3. Verfahren nach Anspruch 1 oder 2, **dadurch gekennzeichnet, dass** die erste Destillationsstufe bei einem Druck von 0,25 bis 1,5 bar durchgeführt wird.

4. Verfahren nach einem der Ansprüche 1 bis 3, **dadurch gekennzeichnet, dass** die dritte Destillationsstufe bei einem Druck von 2,5 bis 8 bar durchgeführt wird.

5. Verfahren nach einem der Ansprüche 1 bis 4, **dadurch gekennzeichnet, dass** die erste Destillationsstufe in einer ersten Destillationskolonne mit mindestens 2 theoretischen Böden, die zweite Destillationsstufe in einer zweiten Destillationskolonne mit mindestens 2 theoretischen Böden und die dritte Destillationsstufe in einer dritten Destillationskolonne mit mindestens 2 theoretischen Böden durchgeführt werden.

6. Verfahren nach Anspruch 5, **dadurch gekennzeichnet, dass** der Abtriebsteil der zweiten Destillationskolonne 50 bis 75 % der Zahl der theoretischen Trennstufen dieser Kolonne aufweist.

7. Verfahren nach einem der Ansprüche 1 bis 6, **dadurch gekennzeichnet, dass** zwischen der ersten und der zweiten Destillationsstufe eine Leichtsieder-Abtrennstufe durchgeführt wird, in der aus dem Strom III Leichtsieder, ausgewählt aus der Gruppe bestehend aus Methylformiat, Methylal, Dimethoxydimethylether und Methanol, abgetrennt werden.

8. Verfahren nach Anspruch 7, **dadurch gekennzeichnet, dass** die Leichtsiederabtrennung bei in einem Druck von 0,1 bis 5,0 bar in einer Destillationskolonne mit mindestens 2 theoretischen Stufen durchgeführt wird.

9. Verfahren nach einem der Ansprüche 1 bis 8, **gekennzeichnet durch** die nachstehende Zusammensetzung der Ströme I - VII und X:
Strom I: 40 bis 80 Gew.-% Formaldehyd, 20 bis 59 Gew.-% Wasser, 1 bis 30 Gew.-% Trioxan;
Strom II: 55 bis 85 Gew.-% Formaldehyd, 15 bis 45 Gew.-% Wasser, 0 bis 5 Gew.-% Trioxan;
Strom III: 3 bis 20 Gew.-% Formaldehyd, 10 bis 30 Gew.-% Wasser, 60 bis 75 Gew.-% Trioxan;
Strom IV: 95 bis 100 Gew.-% Trioxan, 0 bis 5 Gew.-% Wasser und Neben- komponenten;
Strom V: 5 bis 20 Gew.-% Formaldehyd, 15 bis 35 Gew.-% Wasser, 50 bis 75 Gew.-% Trioxan;
Strom VI: 0 bis 1 Gew.-% Formaldehyd, 99 bis 100 Gew.-% Wasser;
Strom VII: 10 bis 55 Gew.-% Formaldehyd, 5 bis 50 Gew.-% Wasser, 5 bis 55 Gew.-% Trioxan.
Strom X: 10 bis 50 Gew.-% Formaldehyd, 10 bis 50 Gew.-% Wasser, 3 bis 40 Gew.-% Trioxan.
wobei die Ströme I, III, V und VII noch bis zu 15 Gew.-% Leichtsieder, ausgewählt aus der Gruppe bestehend aus Methylformiat, Methylal, Dimethoxydimethylether und Methanol, enthalten können.

10. Verfahren zur Herstellung von Trioxan aus einer wässrigen Formaldehydlösung, bei dem ein Strom XI aus einer wässrigen Formaldehydlösung einer Trioxan-Synthesestufe zugeführt und unter sauren Bedingungen umgesetzt wird, wobei der Strom I erhalten wird, und aus dem Strom I nach dem Verfahren gemäß einem der Ansprüche 1 bis 9 Trioxan abgetrennt wird.

11. Verfahren nach Anspruch 10, **dadurch gekennzeichnet, dass** der Strom XI aus einem Strom VIII aus einer wässrigen Formaldehydlösung niedrigerer Formaldehyd-Konzentration durch Aufkonzentrieren in einem Verdampfer erhalten wird.

12. Verfahren nach Anspruch 11, **dadurch gekennzeichnet, dass** der Strom IX der an Formaldehyd abgereicherte Brüdenabzugsstrom des Verdampfers ist.

## Claims

1. A process for removing trioxane from a use stream I of formaldehyde, trioxane and water, by
a) providing a use stream I which comprises formaldehyde as the main component and trioxane and water as the secondary components,
b) feeding the use stream I, a recycle stream V and a recycle stream VII which comprises formaldehyde as the main component and water and trioxane as the secondary components, into a first distillation stage and distilling them at a pressure of from 0.1 to 2.5 bar to obtain a stream II which comprises formaldehyde as the main component and water as the secondary component, and a stream III which comprises trioxane as the main component and water and formaldehyde as the secondary components, and a stream X which comprises water, trioxane and formaldehyde,
c) distilling the stream III, optionally after removing low boilers from the stream III in a low boiler removal stage, in a second distillation stage at a pressure of from 0.2 to 17.5 bar, the pressure in the second distillation stage being from 0.1 to 15 bar higher than the pressure in the first distillation stage, to obtain a stream IV which consists substantially of trioxane and the recycle stream V which comprises trioxane as the main component and water and formaldehyde as the secondary components,
d) feeding the stream X and optionally a stream IX which comprises water as the main component into a third distillation stage and distilling it at a pressure of from 1 to 10 bar to obtain a stream VI which consists substantially of water and a recycle stream VII which comprises formaldehyde as the main component and water and trioxane as the secondary components.

2. The process according to claim 1, wherein the pressure in the second distillation stage is from 1.0 to 10 bar higher than the pressure in the first distillation stage.

3. The process according to claim 1 or 2, wherein the first distillation stage is carried out at a pressure of from 0.25 to 1.5 bar.

4. The process according to any of claims 1 to 3, wherein the third distillation stage is carried out at a pressure of from 2.5 to 8 bar.

5. The process according to any of claims 1 to 4, wherein the first distillation stage is carried out in a first distillation column having at least two theoretical plates, the second distillation stage in a second distillation column having at least 2 theoretical plates and the third distillation stage in a third distillation column having at least two theoretical plates.

6. The process according to claim 5, wherein the stripping section of the second distillation column has from 50 to 75% of the number of theoretical plates of this column.

7. The process according to any of claims 1 to 6, wherein a low boiler removal stage is carried out between the first and the second distillation stage, in which low boilers selected from a group consisting of methyl formate, methylal, dimethoxydimethyl ether and methanol are removed from the stream III.

8. The process according to claim 7, wherein the low boiler removal is carried out at a pressure of from 0.1 to 5.0 bar in a distillation column having at least 2 theoretical plates.

9. The process according to any of claims 1 to 8, **characterized by** the following composition of streams I-VII and X:
stream I: from 40 to 80% by weight of formaldehyde, from 20 to 59% by weight of water, from 1 to 30% by weight of trioxane;
stream II: from 55 to 85% by weight of formaldehyde, 15 to 45% by weight of water, 0 to 5% by weight of trioxane;
stream III: from 3 to 20% by weight of formaldehyde, 10 to 30% by weight of water, 60 to 75% by weight of trioxane;
stream IV: from 95 to 100% by weight of trioxane, 0 to 5% by weight of water and secondary components;
stream V: from 5 to 20% by weight of formaldehyde, 15 to 35% by weight of water, 50 to 75% by weight of trioxane;
stream VI: from 0 to 1% by weight of formaldehyde, 99 to 100% by weight of water;
stream VII: from 10 to 55% by weight of formaldehyde, 5 to 50% by weight of water, 5 to 55% by weight of trioxane;
stream X: from 10 to 50% by weight of formaldehyde, 10 to 50% by weight of water, 3 to 40% by weight of trioxane,
and the streams I, III, V and VII may also contain up to 15% by weight of low boilers selected from the group consisting of methyl formate, methylal, dimethoxydimethyl ether and methanol.

10. A process for preparing trioxane from an aqueous formaldehyde solution, by feeding a stream IX of an aqueous formaldehyde solution to a trioxane synthesis stage and converting it under acidic conditions to obtain the stream I, and removing trioxane from the stream I by the process according to any of claims 1 to 9.

11. The process according to claim 10, wherein the stream XI is obtained from a stream VIII from an aqueous formaldehyde solution of low formaldehyde concentration by concentrating in an evaporator.

12. The process according to claim 11, wherein the stream IX is the formaldehyde-depleted vapor draw stream of the evaporator.

## Revendications

1. Procédé de séparation de trioxane d'un courant de départ I constitué de formaldéhyde, de trioxane et d'eau, selon lequel
a) un courant de départ I, qui contient en tant que composant principal du formaldéhyde et en tant que composants secondaires du trioxane et de l'eau, est préparé,
b) le courant de départ I, un courant de recyclage V et un courant de recyclage VII, qui contient en tant que composant principal du formaldéhyde et en tant que composants secondaires de l'eau et du trioxane, sont introduits dans une première étape de distillation et distillés à une pression de 0,1 à 2,5 bar, un courant II, qui contient en tant que composant principal du formaldéhyde et en tant que composant secondaire de l'eau, et un courant III, qui contient en tant que composant principal du trioxane et en tant que composants secondaires de l'eau et du formaldéhyde, et un courant X, qui contient de l'eau, du trioxane et du formaldéhyde, étant obtenus,
c) le courant III, éventuellement après séparation des composés de point d'ébullition bas du courant III dans une étape de séparation des composés de point d'ébullition bas, est distillé dans une deuxième étape de distillation à une pression de 0,2 à 17,5 bar, la pression lors de la deuxième étape de distillation étant supérieure de 0,1 à 15 bar à la pression lors de la première étape de distillation, un courant IV, essentiellement constitué de trioxane, et le courant de recyclage V, qui contient en tant que composant principal du trioxane et en tant que composants secondaires de l'eau et du formaldéhyde, étant obtenus,
d) le courant X et éventuellement un courant IX, qui contient en tant que composant principal de l'eau, sont introduits dans une troisième étape de distillation et distillés à une pression de 1 à 10 bar, un courant VI, essentiellement constitué d'eau, et un courant de recyclage VII, qui contient en tant que composant principal du formaldéhyde et en tant que composants secondaires de l'eau et du trioxane, étant obtenus.

2. Procédé selon la revendication 1, **caractérisé en ce que** la pression lors de la deuxième étape de distillation est supérieure de 1,0 à 10 bar à la pression lors de la première étape de distillation.

3. Procédé selon la revendication 1 ou 2, **caractérisé en ce que** la première étape de distillation est réalisée à une pression de 0,25 à 1,5 bar.

4. Procédé selon l'une quelconque des revendications 1 à 3, **caractérisé en ce que** la troisième étape de distillation est réalisée à une pression de 2,5 à 8 bar.

5. Procédé selon l'une quelconque des revendications 1 à 4, **caractérisé en ce que** la première étape de distillation est réalisée dans une première colonne de distillation contenant au moins 2 plateaux théoriques, la deuxième étape de distillation dans une deuxième colonne de distillation contenant au moins 2 plateaux théoriques et la troisième étape de distillation dans une troisième colonne de distillation contenant au moins 2 plateaux théoriques.

6. Procédé selon la revendication 5, **caractérisé en ce que** la zone de rectification de la deuxième colonne
de distillation comprend 50 à 75 % du nombre des étapes de séparation théoriques de cette colonne.

7. Procédé selon l'une quelconque des revendications 1 à 6, **caractérisé en ce qu'**une étape de séparation des composés de point d'ébullition bas est réalisée entre la première et la deuxième étape de distillation, lors de laquelle des composés de point d'ébullition bas, choisis dans le groupe constitué par le formiate de méthyle, le méthylal, l'éther diméthoxydiméthylique et le méthanol, sont séparés du courant III.

8. Procédé selon la revendication 7, **caractérisé en ce que** la séparation des composés de point d'ébullition bas est réalisée à une pression de 0,1 à 5,0 bar dans une colonne de distillation contenant au moins 2 étapes théoriques.

9. Procédé selon l'une quelconque des revendications 1 à 8, **caractérisé par** la composition suivante des courants I à VII et X :
courant I : 40 à 80 % en poids de formaldéhyde, 20 à 59 % en poids d'eau, 1 à 30 % en poids de trioxane ;
courant II : 55 à 85 % en poids de formaldéhyde, 15 à 45 % en poids d'eau, 0 à 5 % en poids de trioxane ;
courant III : 3 à 20 % en poids de formaldéhyde, 10 à 30 % en poids d'eau, 60 à 75 % en poids de trioxane ;
courant IV : 95 à 100 % en poids de trioxane, 0 à 5 % en poids d'eau et des composants secondaires ;
courant V : 5 à 20 % en poids de formaldéhyde, 15 à 35 % en poids d'eau, 50 à 75 % en poids de trioxane ;
courant VI : 0 à 1 % en poids de formaldéhyde, 99 à 100 % en poids d'eau ;
courant VII : 10 à 55 % en poids de formaldéhyde, 5 à 50 % en poids d'eau, 5 à 55 % en poids de trioxane ;
courant X : 10 à 50 % en poids de formaldéhyde, 10 à 50 % en poids d'eau, 3 à 40 % en poids de trioxane ;
les courants I, III, V et VII pouvant encore contenir jusqu'à 15 % en poids de composés de point d'ébullition bas, choisis dans le groupe constitué par le formiate de méthyle, le méthylal, l'éther diméthoxydiméthylique et le méthanol.

10. Procédé de fabrication de trioxane à partir d'une solution aqueuse de formaldéhyde, selon lequel un courant XI constitué d'une solution aqueuse de formaldéhyde est introduit dans une étape de synthèse de trioxane et mis en réaction en conditions acides, le courant I étant obtenu, et le trioxane est séparé du courant I par le procédé selon l'une quelconque des revendications 1 à 9.

11. Procédé selon la revendication 10, **caractérisé en ce que** le courant XI est obtenu à partir d'un courant VIII constitué d'une solution aqueuse de formaldéhyde ayant une concentration en formaldéhyde plus faible par concentration dans un évaporateur.

12. Procédé selon la revendication 11, **caractérisé en ce que** le courant IX est le courant de sortie de vapeurs de l'évaporateur appauvri en formaldéhyde.
